Europäisches Patentamt

(19) European Patent Office

Office européen des brevets

(11) Publication number: **0 252 742 B1**

(12) # EUROPEAN PATENT SPECIFICATION

(45) Date of publication of patent specification:
02.10.91 Bulletin 91/40

(51) Int. Cl.$^5$: **C07C 1/20, B01J 29/28**

(21) Application number: 87306084.2

(22) Date of filing: 09.07.87

(54) **Process for conversion of lower aliphatic oxygenates to olefins and aromatics with gallium containing zsm-5 catalyst.**

(30) Priority: 11.07.86 US 884842

(43) Date of publication of application:
13.01.88 Bulletin 88/02

(45) Publication of the grant of the patent:
02.10.91 Bulletin 91/40

(84) Designated Contracting States:
BE DE FR GB IT NL

(56) References cited:
EP-A- 0 074 651
EP-A- 0 074 652
US-A- 4 482 772

(73) Proprietor: MOBIL OIL CORPORATION
3225 Gallows Road
Fairfax, Virginia 22037-0001 (US)

(72) Inventor: Chu, Cynthia Ting-Wah
18 Indian Run Road
Princeton Junction, NJ 08550 (US)

(74) Representative: Jones, Alan John et al
CARPMAELS & RANSFORD 43 Bloomsbury
Square
London, WC1A 2RA (GB)

## Description

Recently, efforts to prepare olefins from methanol have become increasingly important. Methanol can be readily produced from coal, via coal gasification and the production of synthesis gas, with the aid of well-tried technology. If it were possible to convert methanol to lower olefins in an economical manner, the further processing methods which are conventional in the chemical industry today, and employ coal as a raw material, could also be preserved. In the past few years, processes have, therefore, been developed with the object of preparing olefins from methanol, dimethyl ether or mixtures thereof.

US-A-3,894,106, 3,894,107, and 3,907,915, respectively, disclose the conversion of alcohols, ethers, carbonyls, or mixtures thereof to olefinic and aromatic hydrocarbons by contact with a catalyst comprising a crystalline aluminosilicate zeolite having a silica-to-alumina ratio of at least about 12 and a Constraint Index of about 1 to 12.

The conversion of methanol and dimethyl ether to olefinic and aromatic hydrocarbons is described in US-A-3,911,041.

DE-A-2,615,150 relates to a process for the preparation of olefins from a methanol feed using a ZSM-5 aluminosilicate zeolite.

US-A-4,361,715 relates to the catalytic conversion of lower molecular weight oxygenates to olefins over a bed of aluminosilicate zeolite catalyst. Gallium can be incorporated into the framework of the zeolite, but the zeolite does not have the ZSM-5 framework structure.

US-A-4,401,637 discloses a process for preparing olefins from methanol over a catalyst bed of a modified crystalline isotactic zeolite comprising framework gallium and an organic amine of low basicity, but the catalysts differ from ZSM-5 type catalysts.

UA-A-4,582,949 discloses a two-stage conversion process for the preparation of aromatic hydrocarbons from olefins over metallosilicate catalysts. A crystalline gallium silicate catalyst having a silicon dioxide to gallium trioxide ratio of between 25 and 250 and a specific x-ray powder diffraction pattern is employed in the second stage.

EP-A 74652 discloses that offretite containing gallium or indium introduced during synthesis is superior to the metal-free material as catalyst for conversion of methanol to hydrocarbons.

A new catalytic process has now been discovered for the conversion of lower aliphatic $C_1$-$C_4$ oxygenates by a series of dehydration (or decarbonylation) and dehydrogenation reactions employing a medium pore acidic crystalline gallium-containing siliceous zeolite of ZSM-5 framework. It has been found that gallium containing ZSM-5 zeolites have superior catalytic activity in the production of $C_2$-$C_5$ olefins and aromatics, useful as intermediates in the preparation of gasoline/distillate or as valuable chemical feedstock. The process gives much lower yield of less valuable $C_1$-$C_5$ paraffins than a similar process employing a standard aluminosilicate H-ZSM-5 catalyst.

According to the present invention a process for the conversion of a $C_1$ to $C_4$ aliphatic oxygenate feed to $C_2$-$C_5$ olefins, with augmented yield of aromatics and diminished yield of C1-C5 alkanes, comprises contacting said feed, at a temperature between 350 and 575°C and a pressure of 10 to 3500 kPa, with crystalline acidic zeolite ZSM-5 having a silica/alumina mole ratio of at least 500 and containing gallium of which a portion is in tetrahedral coordination in the framework of the zeolite, and withdrawing a product containing light olefins, aromatics, and less than 10% wt. of said alkanes. Preferably the contacting is performed at 370 to 500°C and the ratio of product light olefins to product aromatics is at least 3:1. Suitably the contacting is effected at WHSV of 0.1 to 10. A particularly contemplated feed is methanol and/or dimethyl ether.

The upgrading of lower molecular weight $C_1$-$C_4$ oxygenates, such as alcohols, ethers, ketones and their acetals, and carboxylates is achieved in an efficient manner by contacting the oxygenates under reaction conditions with a medium pore acidic crystalline gallium containing ZSM-5 type catalyst having at least a portion of zeolitic gallium in tetrahedral coordination therein to provide Bronsted acid sites.

In a preferred embodiment, the process comprises: maintaining a reaction zone containing a crystalline acidic gallium containing ZSM-5 catalyst; passing a feedstock comprising methanol, dimethylether or mixtures thereof through the reaction zone at conditions of elevated temperature; and withdrawing a product rich in light olefins and aromatics. Preferably, the ratio of light olefins ($C_2$-$C_5$) to aromatic hydrocarbons is at least about 3:1. The amount of less desirable paraffinic hydrocarbons is reduced to a minimum by the process, producing less than 10 wt.% $C_1$-$C_5$ alkanes. At higher conversion temperatures, the ratio of aromatics to saturates is at least 2:1, and preferably at least 3:1.

The gallium zeolite catalyst can be prepared by various methods; for example: by a cocrystallization procedure, vapor phase treatment, or by employing an alkaline medium. ZSM-5 zeolite having a silica-to-alumina ratio of at least 500 can be directly synthesized by known methods, or prepared from a zeolite having a lower silica-to-alumina ratio by steaming, dealuminizing, or framework exchange (lattice substitution).

The prepared gallium containing zeolite, in a preferred embodiment, has the framework structure of ZSM-5 with Bronsted acid sites provided by tetrahedrally coordinated gallium in the framework structure. The amount of alumina in the zeolite is less than 0.5 wt.%. The tetrahedral gallium of the framework may be indicated by ammonium exchange capacity and Nuclear Magnetic Resonance (NMR) signal. Part of the gallium associated with the zeolite may, however, not be in tetrahedral coordination in the framework, since it has been observed that such non-framework gallium assists in the minimisation of light alkane production.

The Examples which follow illustrate the process according to the invention.

## PREPARATION OF CATALYST

A gallium containing zeolite was synthesized by lattice substitution from ZSM-5 having a silica-to-alumina ratio of 26,000, which has been calcined at 538°C (1000°F). A teflon bottle containing 2g of the calcined ZSM-5 and 4g of $GaCl_3$ in 30 cc of $H_2O$ was sealed and heated at 150°C for 18 hours. The product was washed and ion exchanged with 1N ammonium nitrate, followed by calcination in air. It contained 2.2% gallium.

## EXAMPLE 1

A feedstock of methanol was reacted over a catalyst bed of gallium containing ZSM-5 zeolite prepared according to Example 1, at 500°C (932°F), a pressure of 101 kPa (1 atmosphere) and a weight hourly space velocity (WHSV) of the methanol feed of 1.

The results are given in Column A of Table 1. At 98% conversion, the process produces a mixture of hydrocarbons consisting of 62 wt.% $C_2$-$C_5$ olefins, 20 wt.% aromatics, and only 6 wt.% $C_2$-$C_5$ light paraffins.

## EXAMPLE 2

Example 1 was repeated at a temperature of 370°C.

The results are given in Column B of Table 1. At 7% conversion, the process produces a mixture of hydrocarbons consisting of 81 wt.% $C_2$-$C_5$ olefins and 20 wt.% $C_1$-$C_5$ paraffins.

## EXAMPLE 3 (Comparative)

A feedstock of methanol was reacted over a catalyst bed of aluminosilicate ZSM-5 zeolite having a silica-to-alumina ratio of 1,670 and an acid-cracking (alpha) value of 5. The reaction was conducted under the conditions of Example 1.

The results of the experiment are given in Column C of Table 1.

## EXAMPLE 4 (Comparative)

A feedstock of methanol was reacted over a catalyst bed of aluminosilicate ZSM-5 zeolite having a silica-to-alumina ratio of 500 and an acid-cracking (alpha) value of 12. The reaction was conducted under the conditions of Example 1.

The results of the experiment are given in Column D of Table 1.

## EXAMPLE 5 (Comparative)

A feedstock of methanol was reacted over a catalyst bed of aluminosilicate ZSM-5 zeolite having a silica-to-alumina ratio of 70 and an acid-cracking (alpha) value of 150. The reaction was conducted under the conditions of Example 1.

The results of the experiment are given in Column E of Table 1.

## TABLE 1

| Column | A | B | C | D | E |
|---|---|---|---|---|---|
| Catalyst Type | Ga/ZSM-5 | Ga/ZSM-5 | ZSM-5 | ZSM-5 | ZSM-5 |
| $SiO_2/Al_2O_3$ Ratio | 26,000 | 26,000 | 1,670 | 500 | 70 |
| $C_1$-$C_5$ product | 5.89 | 19.48 | 6.59 | 10.91 | 37.56 |
| $C_2$= - $C_5$= product | 62.04 | 80.82 | 79.81 | 71.67 | 38.01 |
| $C_6$+ PON * product | 11.54 | ------- | 5.21 | 4.34 | 1.7 |
| Aromatics product | 20.53 | ------- | 8.39 | 13.06 | 22.72 |
| % conversion | 98.14 | 7 | 99+ | 99+ | 99+ |

* Paraffins, olefins and naphthenes

The results indicated in Table 1 illustrate the effectiveness of the inventive process for producing olefinic and aromatic hydrocarbons from oxygenate feed with a minimum formation of light paraffins.

## Claims

1. A process for the conversion of a C1 to C4 aliphatic oxygenate feed to C2-C5 olefins, with augmented yield of aromatics and diminished yield of C1-C5 alkanes, which comprises contacting said feed, at a temperature between 350 and 575°C and a pressure of 10 to 3500 kPa, with crystalline acidic zeolite ZSM-5 having a silica/alumina mole ratio of at least 500 and containing gallium of which a portion is in tetrahedral coordination in the framework of the zeolite.

2. A process according to claim 1 wherein the contacting is performed at 370 to 500°C.

3. A process according to claim 1 or claim 2 wherein the contacting is effected at WHSV of 0.1 to 10.

4. A process according to any preceding claim wherein said feed is methanol and/or dimethyl ether.

## Patentansprüche

1. Verfahren zur Umwandlung einer aliphatischen sauerstoffhaltigen $C_1$-$C_4$-Zufuhr in $C_2$-$C_5$ Olefine mit erhöhter Ausbeute an Aromaten und verringerter Ausbeute an $C_1$-$C_5$-Alkanen, das den Kontakt dieser Zufuhr bei einer Temperatur zwischen 350°C und 575°C und einem Druck von 10 - 3500 kPa mit einem kristallinen sauren Zeolith ZSM-5 umfaßt, der ein Siliciumdioxid/Aluminiumoxid-Molverhältnis von mindestens 500 aufweist und Gallium enthält, von dem ein Teil im Gitter des Zeolith tetraedisch gebunden ist.

2. Verfahren nach Anspruch 1, worin der Kontakt bei 370°C - 500°C durchgeführt wird.

3. Verfahren nach Anspruch 1 oder 2, worin der Kontakt bei einer WHSV von 0,1 bis 10 durchgeführt wird.

4. Verfahren nach einem der vorstehenden Ansprüche, worin die Zufuhr Methanol und/oder Dimethylether ist.

## Revendications

1. Un procédé pour la conversion d'une charge de composés aliphatiques en $C_1$ à $C_4$ oxygénés en oléfines en $C_2$-$C_5$ avec un rendement plus élevé en aromatiques et plus faible en alcanes en $C_1$ à $C_5$, qui comprend la mise en contact de ladite charge, à une température comprise entre 350 et 575°C et à une pression de 10 à 3 500 kPa, avec une zéolite cristalline acide du type ZSM-5 ayant un rapport molaire silice/alumine d'au moins 500 et contenant du gallium, une partie dudit gallium étant tétracoordonnée dans la structure de ladite zéolite.

2. Le procédé selon la revendication 1, dans lequel l'étape de mise en contact est mise en oeuvre à une température comprise entre 370 et 500°C.

3. Le procédé selon la revendication 1 ou 2, dans lequel l'étape de mise en contact est mise en oeuvre à

4

une vitesse spatiale horaire pondérale (VSHP) de 0,1 à 10.

4. Le procédé selon l'une quelconque des revendications précédentes, dans lequel ladite charge est du méthanol et/ou du diméthyléther.